# EUROPÄISCHE PATENTSCHRIFT

(11) **EP 3 265 290 B1**
(45) Veröffentlichungstag und Bekanntmachung des Hinweises auf die Patenterteilung: **30.10.2019**
(21) Anmeldenummer: 17713230.5
(22) Anmeldetag: 22.03.2017
(51) Int. Cl.: B29C 55/06, B29C 55/18, B32B 27/20, B32B 27/08, B29C 55/02, B29C 44/56, B29C 71/02, B32B 5/02, B32B 7/04, B32B 7/12, B32B 27/12, B32B 27/18, B32B 27/28, B32B 27/30, B32B 27/32, B29K 23/00, B29K 105/04, B29K 105/16, B29C 55/00, A61F 13/15

(54) **VERFAHREN ZUR HERSTELLUNG EINER MEHRLAGIGEN FOLIENBAHN UND MEHRLAGIGE FOLIENBAHN**
METHOD FOR PRODUCING A MULTI-LAYER FILM WEB AND MULTI-LAYER FILM WEB
PROCEDE DE FABRICATION D'UNE BANDE DE FILM MULTICOUCHES ET BANDE DE FILM MULTICOUCHES

(30) Priorität: 22.03.2016 EP 16161715
(43) Veröffentlichungstag der Anmeldung: 10.01.2018
(73) Patentinhaber: RKW SE, 67227 Frankenthal (DE)
(72) Erfinder: BÖRMANN, Ludwig, 83547 Babensham (DE)
(74) Vertreter: Lang, Johannes
(86) Internationale Anmeldenummer: PCT/EP2017/056837
(87) Internationale Veröffentlichungsnummer: WO 2017/162746

(56) Entgegenhaltungen:
- EP-A1- 0 133 188
- EP-A1- 2 353 844
- EP-A1- 2 565 013
- EP-A1- 2 952 330

## Beschreibung

Die Erfindung betrifft ein Verfahren zur Herstellung einer mehrlagigen Folienbahn, eine damit hergestellte Folienbahn sowie ihre Verwendung, beispielsweise im Hygienesektor.

Im Zuge der Umweltdiskussion zur Ressourcenschonung und Nachhaltigkeit wird es auf dem Gebiet der Folien, vor allem von Folien für Wegwerfprodukte aus dem Hygienesektor, von immer größerer Bedeutung, noch dünnere Folien als bisher herzustellen, um Rohstoffe einzusparen.

Aus der EP-A-0 768 168 und der EP-A-1 716 830 sind Verfahren zur Herstellung von im Hygienebereich einsetzbaren Folien bekannt. An solche Hygienefolien werden aufgrund ihres Einsatzbereichs mehrere Anforderungen gestellt. Sie sollen flüssigkeitsdicht sein und über bestimmte haptische Eigenschaften verfügen, wie Weichheit, Geschmeidigkeit, raschelarmes Verhalten und textiler Griff. Folien im Hygienebereich sollen einen weichen, stoffähnlichen Griff besitzen. Insbesondere bei ihrer Verwendung für Inkontinenzprodukte soll die Geräuschentwicklung möglichst gering sein, d.h. die Folien sollen raschelarm sein. In Verbindung mit einem geringen Glanzgrad ergibt dies eine sehr textile Folie, was im Hygienebereich erwünscht ist. Hinzu kommt, dass in den letzten Jahren die in Windeln und Inkontinenzprodukten enthaltenen Saugkörper immer dünner geworden sind, was insbesondere durch die Verwendung von Superabsorber-Polymeren ermöglicht wurde. Diese Superabsorber-Polymere werden in Form grobkörniger Pulver eingesetzt, und die Hygienefolien müssen eine derartige Festigkeit besitzen, dass ein Durchstoßen der Folie durch die einzelnen Körner, z.B. bei Belastung durch Hinsetzen oder sonstige Bewegung des Trägers, mit hoher Sicherheit vermieden wird. Eine Bildung von Löchern (*"Pinholes*") durch Superabsorber-Polymere und ein Platzen der Fertigfolienprodukte in den Verpackungseinheiten müssen vermieden werden. Eine weitere Anforderung an Hygienefolien besteht in einer Mindestzugfestigkeit, die für eine Verarbeitung der Folienbahnen auf den schnell laufenden Maschinen (Konverter) der Hersteller von z.B. Windeln und Damenbinden erforderlich ist. Diese Mindestzugfestigkeit wird in der Regel für 5%, 10 % oder 25 % Dehnung in Maschinenrichtung (MD) oder Querrichtung (CD) angegeben. Darüber hinaus sollen Folien für Hygieneanwendungen bestimmte Festigkeiten aufweisen, beispielsweise einlagige Backsheets eine Längsreißfestigkeit von wenigstens 10 N/inch und eine Querreißfestigkeit von wenigstens 5 N/inch. Wird das Backsheet mit einem Vlies laminiert, soll die Längsreißfestigkeit wenigstens 5 N/inch und die Querreißfestigkeit wenigstens 2 N/inch betragen.

Bekannt ist auch die Verwendung von Laminaten aus Folie und Vlies. Eine Herstellung derartiger Laminate ist in der WO 2006/024394 beschrieben, bei der eine Ausgangsfolienbahn aus thermoplastischem Polymermaterial zusammen mit einer Ausgangsvliesbahn, deren Schmelzpunkt oberhalb des Kristallitschmelzpunktes des Polymermaterials liegt, auf eine Temperatur über dem Kristallitschmelzpunkt des Polymermaterials und unter dem Schmelzpunkt der Ausgangsvliesbahn erwärmt und das gebildete Laminat durch einen gekühlten Walzenspalt geführt und dabei auf eine Temperatur unterhalb des Kristallitschmelzpunktes der Ausgangfolienbahn gekühlt wird.

In der EP-A-0 768 168 wird eine Ausgangsfolienbahn aus thermoplastischem Polymermaterial bis zum schmelzeflüssigen Zustand des Polymermaterials erwärmt und danach durch einen gekühlten Walzenspalt geführt. In der EP-A-1 716 830 wird ein Verfahren mit Erwärmen des Polymermaterials und anschließendes Führen durch einen gekühlten Walzenspalt mit einer Ausgangsfolienbahn durchgeführt, die ein thermoplastisches Polymermaterial mit einer Polyethylen-Matrix enthält, in der 1 bis 70 Gewichtsteile Polypropylen, bezogen auf 100 Gewichtsteile Polyethylen-Matrix enthalten sind. Hierbei wird die Erwärmung der Ausgangsfolienbahn bis zum schmelzeflüssigen Zustand des Polyethylen-Matrixmaterials, jedoch nicht bis zum schmelzeflüssigen Zustand des Polypropylens durchgeführt.

Um die Dicke von Folien zu verringern, ist aus dem Stand der Technik das Recken oder Verstrecken von Folienbahnen bekannt. Beispielsweise beschreibt die EP-A-2 565 013 ein Verfahren zum Recken einer Ausgangsfolienbahn aus thermoplastischem Polymermaterial, welches eine niedrig schmelzende Polymerkomponente und eine hoch schmelzende Polymerkomponente enthält. Das Verfahren umfasst das Erwärmen der Ausgangsfolienbahn bis zum teilweise schmelzflüssigen Zustand, bei dem sich eine niedrig schmelzende Polymerkomponente im schmelzeflüssigen Zustand und eine hoch schmelzende Polymerkomponente nicht im schmelzeflüssigen Zustand befindet, durch eine Heizwalze, und das Abkühlen der teilweise geschmolzenen Folienbahn durch Führen durch einen gekühlten Walzenspalt, wobei die Folienbahn zwischen der Heizwalze und dem gekühlten Walzenspalt gereckt wird.

Um Rohstoffe einzusparen, ist allgemein der Zusatz von Füllstoffen zu Folien bekannt. Werden gefüllte Folien gereckt, werden sie atmungsaktiv. Zur Herstellung von atmungsaktiven Folien werden Folien mit etwa 60 % inertem Material gefüllt und nach dem Extrudieren einem Reckprozess (meist einem Recken in Maschinenrichtung) unterzogen, um die Folie atmungsaktiv zu machen. Als Füllstoff wird üblicherweise Kreide (CaCO₃) in einer Partikelgröße von 0,8-2µm eingesetzt. Beim Reckprozess werden die elastischen polymeren Anteile der Folie gedehnt und es entstehen Poren am Rand der Kreidekörner hin zur Polymermatrix. Durch Streuungen der Kreidepartikelgrößen (bis 12 µm und größer) können auch Porengrößen entstehen, die zu Dichtigkeitsproblemen führen können. Dieses Problem wird verstärkt, wenn zur Herstellung möglichst dünner, atmungsaktiver Folien beim Recken relativ hohe Reckgrade von z.B. 2:1 bis 3:1 nötig sind. Teilweise zeigen in Maschinenrichtung gereckte Folien auch geringe Sicherheit gegen Undichtigkeiten (sogenanntes Leakage). Zudem besteht die Gefahr, dass an einigen Stellen in der Folie die erzeugten Poren zu groß werden (>1µm) und damit ein Problem der Durchnässung entsteht (d.h. Flüssigkeitsdurchstoßfestigkeitswerte (Liquid Impact Werte) größer als 3 g/m² vorliegen). Erwünscht sind zum Teil Werte für die Flüssigkeitsdurchstoßfestigkeit von kleiner 2 g/m² oder gar kleiner 1,5 g/m².

Verfahren zur Herstellung atmungsaktiver Folien sind beispielsweise aus der EP 0 921 943 B1, EP 1 226 013 B1, EP 1 711 330 B1 und GB 2 364 512 B bekannt. Atmungsaktive Folien müssen den obigen Anforderungen an mechanische Eigenschaften wie ungefüllte Folien genügen und weiterhin flüssigkeitsdicht sein. Im Zuge der Ressourcenschonung und Nachhaltigkeit werden auch bei ihnen geringe Dicken angestrebt.

Aus der EP 2 952 330 A1 sind eine Blasfolienanlage und ein Verfahren zum Herstellen einer Blasfolienbahn bekannt, wobei nach dem Abzug ein Längsstrecken der erzeugten Doppellagenbahn vorgesehen ist. Gemäß der EP 2 952 330 A1 wird die Folie oberhalb des Abzugs erwärmt und dann mechanisch behandelt.

Bekanntlich haben Folien einen sogenannten Rückerinnerungseffekt. Das bedeutet, dass Folien, die beispielsweise bei 80°C gereckt und bei 100°C anschließend einem Annealing unterzogen wurden, bei nochmaligen Erreichen dieser Temperaturen, z. B. bei sehr heißen Hotmeltklebstoffen (ca. 160°C) im Konverter, zu schrumpfen versuchen. Dieses Problem tritt gerade bei kreidegefüllten Folien wegen ihrer guten Wärmeleitfähigkeit und bei besonders dünnen Folien auf. Bei zu hoher Temperaturbelastung bzw. zu geringer Foliendicke können dann sehr schnell unerwünschte Löcher (sogenannter Burn-Through-Effekt) entstehen.

Üblicherweise werden heutzutage atmungsaktive Folien nach dem Reckprozess für einige Tage zwischengelagert und die Nachkristallisation abgewartet, bevor es zur Weiterverarbeitung, z.B. Bedrucken, kommt, da die Folien nachschrumpfen können. Soll also eine Folie bedruckt werden, muss nach dem Reckvorgang und vor dem Druckvorgang eine Kristallisationszeit von etwa 1 bis 3 Tagen abgewartet werden. Dieser Vorgang verursacht sehr hohe Kosten und behindert ein Inline-Drucken der Folien.

Gefüllte und gereckte Folien neigen auf den fertigen Rollen zum Blocking. Ein Blocking bedeutet, dass die Folienlagen durch Nachschrumpf derart aneinander haften, dass beim Abrollen Schwierigkeiten auftreten z.B. dass die Folie sogenannte Spiralrisse zeigt. Bei Spiralrissen haftet die Folie partiell auf der darunterliegenden Folienlage. Dies führt zu Abrissen der Folie beim Abrollvorgang, wovon die Bereiche in der Nähe des Schnittspiegels besonders betroffen sind. Ein Blocking stellt vor allem beim Abrollen von dünnen Folien ein Problem dar.

In Maschinenrichtung (MD) gereckte Folien haben eine geringe Durchstoßfestigkeit gegenüber scharfkantigen Superabsorber-Körnern, die häufig in Hygieneprodukten zur Flüssigkeitsaufnahme verwendet werden. Da diese Körner oft in direktem Kontakt mit der Folie stehen, kann es zu Pinholes und Undichtigkeiten (Leakage) im Fertigprodukt kommen. Zudem zeigen gefüllte und MD-gereckte Folien eine geringe MD-Weiterreißfestigkeit und eine geringe MD-Einreißfestigkeit. Geringste Verletzungen auf der Rollenstirnseite oder ein geringes Blocking der Folie auf der Rolle können zum Einreißen und Weiterreißen führen, wodurch sich Spiralrisse bilden.

Der Reckprozess verstärkt generell die Unterschiede von Dick- und Dünnstellen in der Folie und kann zusätzlich zur Randverdickung führen, auch "Neck In" genannt. Beide Effekte verursachen auf den fertigen Rollen sogenannte Kolbenringe. Das bedeutet, dass beim Abrollen dieser Rollen lange Kanten oder Durchhänger in der Folie entstehen, die wiederum zu großen Schwierigkeiten beim Konvertierprozess führen können (z.B. CD-Versatz der Folie). Hohe Reckgrade verstärken die Randverdickung (Neck In) der Folie, den Nachschrumpf der Folie nach dem Reckprozess und eine sehr geringe Weitereißfestigkeit der Folie in Maschinenrichtung. Oft werden die Rollen auch in sogenannten Mutterrollen zwischengelagert und erst nach erfolgtem Nachschrumpf (Kristallisation) einem Schneidumroller zugeführt, in dem sie dann auf gewünschte Kundenbreite zugeschnitten werden. Der Nachschrumpf der atmungsaktiven Folien kann auf den fertigen Rollen eine erhebliche Lagenpressung auslösen, welche wiederum zwischen den Folienlagen ein Blocking auslösen und zu Spiralrissen beim Abrollen der Folie führen kann.

Insbesondere bei Backsheets (Unterlagenschichten für Windeln und Hygieneprodukte) lösen Randverdickungseffekte, wie Durchhänger und lange Folienkanten, große Probleme beim Einlaufen in die Konverter aus, da zum einen der Reckprozess in Maschinenrichtung sehr stark vorhandene Dick- und Dünnstellen verstärkt und zum anderen ein Versatz der Folien in Querrichtung (CD) eintreten kann, was letztendlich zu einem Stillstand des Konverters führen kann. Daher ist ein planliegendes Einlaufen von Backsheets in Konvertern von großer Bedeutung.

Hinzu kommt, dass mehr Fehlstellen oder Löcher in den Folien auftreten können, je dünner die Folien werden. Solche Löcher oder Fehlstellen können beispielsweise mit einer CCD (*charge-coupled* device)-Kamera erfasst werden. Zunehmend werden Folien mit Löchern oder Fehlstellen von Herstellern von Fertigfolienprodukten nicht mehr akzeptiert und stellen somit Ausschuss dar. Zudem erlaubt die sich ständig verbessernde Messtechnik ein einfacheres Erkennen von Löchern oder Fehlstellen. Es besteht deshalb ein Bedarf an einem Verfahren, dass die Anzahl der Löcher oder Fehlstellen in dünnen Folien verringert.

Zur Lösung einer oder mehrerer dieser Probleme schlägt die vorliegende Erfindung vor, zwei Ausgangsfolienbahnen zusammen bis zu ihrem jeweils teilweise schmelzflüssigen Zustand zu erwärmen und anschließend die erhaltene mehrlagige Folienbahn in einem gekühlten Walzenspalt schnell abzukühlen. Durch das Übereinanderlegen der zwei Ausgangsfolienbahnen wird die Anzahl der Löcher oder Fehlstellen in der mehrlagigen Folienbahn verringert, da es sehr unwahrscheinlich ist, dass zwei Fehlstellen oder Löcher beim Erwärmen in den teilweise schmelzflüssigen Zustand exakt übereinander zu liegen kommen. Durch den teilweise schmelzflüssigen Zustand und das anschließende Abkühlen der mehrlagigen Folienbahn werden die Eigenschaften der Folie signifikant verbessert und damit die obengenannten Probleme gelöst. Die mehrlagige Folienbahn kann zwischen dem Heizzylinder und dem gekühlten Walzenspalt gereckt werden. Dadurch wird das Flächengewicht oder die Dicke der durch das Aufeinanderlegen von zwei Folienbahnen erhaltenen Folienbahn wieder verringert. Somit kann das Flächengewicht oder die Dicke der dickeren Folienbahn wieder kompensiert werden.

Fehlstellen oder Löcher in einer Folie bedeuten vorliegend Fehlstellen oder Löcher ab einem Durchmesser von 0,5 mm. Solche Fehlstellen oder Löcher sind z.B. sichtbar, wenn die Folie gegen Licht gehalten wird. Sie können mit einer CCD (*charge-coupled* device)-Kamera oder einem CMOS-Kamerasystem erfasst werden. Fehlstellen oder Löcher sind wesentlich größer als Mikroporen. Der Begriff "Mikroporen" oder "mikroporös" bedeutet vorliegend im wesentlichen Poren einer Größe von 0,1 bis 5 µm. Im wesentlichen bedeutet hierbei, dass wenigstens 90 % der Poren, bevorzugt 95 %, stärker bevorzugt 99% der Poren, oder gar 99,9% der Poren eine Größe 0,1 bis 5 µm haben und die restlichen Poren etwas größer sind, im allgemeinen bis zu 15 µm.

Somit betrifft die Erfindung ein Verfahren zur Herstellung einer mehrlagigen Folienbahn aus wenigstens zwei Ausgangsfolienbahnen aus thermoplastischem Polymermaterial, wobei jede Ausgangsfolienbahn wenigstens eine niedrig schmelzende Polymerkomponente und wenigstens eine hoch schmelzende Polymerkomponente umfasst, wobei das Verfahren folgende Schritte umfasst: Herstellen der wenigstens zwei Ausgangsfolienbahnen durch Blasextrusion, Cast-Extrusion, oder eine Kombination von Blasextrusion und Cast-Extrusion; Führen der wenigstens zwei Ausgangsfolienbahnen bis zu deren teilweise schmelzflüssigen Zustand zusammen über wenigstens eine Heizwalze, wobei sich in jeder Ausgangfolienbahn die wenigstens eine niedrig schmelzende Polymerkomponente im schmelzflüssigen Zustand befindet und die wenigstens eine hoch schmelzende Polymerkomponente nicht im schmelzflüssigen Zustand befindet; und Führen der mehrlagigen, teilweise geschmolzenen Folienbahn durch einen gekühlten Walzenspalt.

Die Ausgangsfolienbahnen können gleich oder verschieden sein. Es können zwei, drei, vier oder mehr Ausgangsfolienbahnen verwendet werden. Vorzugsweise werden zwei Ausgangsfolienbahnen verwendet. Die Ausgangsfolienbahnen können zwei Ausgangsfolienbahnen sein, die durch Blasextrusion hergestellt sind. Beispielsweise können sie hergestellt werden, indem ein Blasfolienschlauch hergestellt wird, der Schlauch flachgelegt wird, der Schlauch gegebenenfalls an den beiden Seiten aufgetrennt oder aufgeschlitzt wird, und anschließend die beiden Folienbahnen getrennt oder zusammen der Heizwalze zugeführt werden.

In bevorzugten Ausführungsformen des erfindungsgemäßen Verfahrens umfasst jede Ausgangsfolienbahn 15 bis 85 Gew.-% niedrig schmelzende Polymerkomponente und 85 bis 15 Gew.-% hoch schmelzende Polymerkomponente, bezogen auf 100 Gew.-% niedrig und hoch schmelzende Polymerkomponente.

In anderen bevorzugten Ausführungsformen des erfindungsgemäßen Verfahrens umfasst jede Ausgangsfolienbahn wenigstens ein Polyethylen als niedrig schmelzende Polymerkomponente und wenigstens ein Polypropylen als hoch schmelzende Polymerkomponente.

Bei der Durchführung des Verfahrens wird bevorzugt jede Ausgangsfolienbahn auf 5 bis 20 °C unterhalb des Kristallitschmelzpunktes der wenigstens einen hoch schmelzenden Polymerkomponente erwärmt.

In beispielhaften Ausführungsformen des Verfahrens werden die den gekühlten Walzenspalt bildenden Walzen bei einer höheren Geschwindigkeit als die wenigstens eine Heizwalze angetrieben werden. Dadurch wird die mehrlagige Folienbahn zwischen der wenigstens einen Heizwalze und dem gekühlten Walzenspalt gereckt. Beispielhafte Reckverhältnisse betragen wenigstens 1:1,2, bevorzugt wenigstens 1:1,5, stärker bevorzugt wenigstens 1.2.

Bevorzugt wird die mehrlagige Folienbahn im gekühlten Walzenspalt einer Abkühlung auf wenigstens 10 bis 30 °C unterhalb des Kristallitschmelzpunktes der wenigstens einen niedrig schmelzenden Polymerkomponente jeder Ausgangsfolienbahn unterworfen wird. Vorzugsweise wird der gekühlte Walzenspalt durch eine Prägewalze und eine Gummiwalze gebildet. Die Folienbahn kann nach dem Abkühlen bedruckt werden.

In bevorzugten Ausführungsformen enthält wenigstens eine Ausgangsfolienbahn Füllstoff. Bevorzugt enthalten zwei Ausgangsfolienbahnen Füllstoff. Beispielhafte Mengen für Füllstoff sind 10 bis 90 Gew.-%, bevorzugt 20 bis 80 Gew.-%, bezogen auf jeweils 100 Gew.-% der Ausgangsfolienbahn.

In beispielhaften Ausführungsformen ist wenigstens eine Ausgangsfolienbahn mikroporös. Die mikroporöse Ausgangsfolienbahn kann atmungsaktiv oder nicht-atmungsaktiv sein.

In weiteren beispielhaften Ausführungsformen wird wenigstens eine Ausgangsfolienbahn im Verlauf ihrer Herstellung in Maschinen- oder Querrichtung oder in Maschinen- und Querrichtung gereckt.

Außerdem betrifft die Erfindung die mit den beschriebenen Verfahren hergestellten mehrlagigen Folienbahnen, beispielsweise mit einem Flächengewicht von 1 bis 30 g/m², insbesondere von 5 bis 25 g/m², bevorzugt von 7 bis 20 g/m², stärker bevorzugt von 10 bis 20 g/m², sowie deren Verwendung, insbesondere im Hygiene- oder Medikalbereich, zum Beispiel für Backsheets in Windeln, für Betteinlagen oder Damenbinden. Daneben betrifft die Erfindung die Verwendung der hergestellten Folienbahnen im Baubereich, z. B. als Abdeckfolien, oder als Automobilschutzfolien.

Bevorzugte Ausführungsformen der Erfindung sind in der nachfolgenden Beschreibung, dem Beispiel, der Figur und den Ansprüchen beschrieben.

Bei den Figuren zeigt:

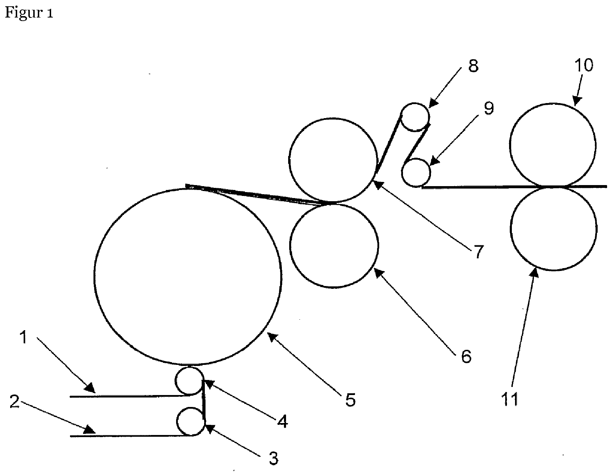

Figur 1 eine bevorzugte Ausführungsform zur Durchführung des erfindungsgemäßen Verfahrens.

In der vorliegenden Erfindung beziehen sich die angegebenen Schmelzpunkte, Schmelzbereiche und Kristallitschmelzpunkte auf eine Bestimmung gemäß DSC (Differential Scanning Calorimetry).

Erfindungsgemäß enthält oder umfasst jede Ausgangsfolienbahn wenigstens eine niedrig schmelzende Polymerkomponente und wenigstens eine hoch schmelzende Polymerkomponente. Mit anderen Worten, jede Ausgangsfolienbahn enthält eine oder mehrere niedrig schmelzende Polymerkomponente(n) und eine oder mehrere hoch schmelzende Polymerkomponente(n). Das Gleiche bedeuten die in der vorliegenden Erfindung weiterhin verwendeten Begriffe "eine niedrig schmelzende Polymerkomponente" und "eine hoch schmelzende Polymerkomponente", d.h. auch sie umfassen eine oder mehrere niedrig bzw. hoch schmelzende(n) Polymerkomponente(n). Vorzugsweise enthält jede Ausgangsfolienbahn eine, bevorzugt zwei, niedrig schmelzende Polymerkomponente(n). Bevorzugt enthält sie eine, insbesondere zwei, hoch schmelzende Polymerkomponente(n). In anderen Ausführungsformen der Erfindung enthält sie bevorzugt drei niedrig schmelzende Polymerkomponenten und/oder drei hoch schmelzende Polymerkomponenten. Ob ein Polymermaterial der Ausgangsfolienbahn zur niedrig schmelzenden Polymerkomponente oder zur hoch schmelzenden Polymerkomponente gehört, bestimmt sich erfindungsgemäß nach dem jeweiligen Kristallitschmelzpunkt, Schmelzpunkt oder Schmelzbereich des Polymermaterials im Verhältnis zur Erwärmungstemperatur. Bei gegebener Erwärmungstemperatur werden die schmelzeflüssigen Polymermaterialien der niedrig schmelzenden Polymerkomponente und die nicht-schmelzeflüssigen Polymermaterialien der hoch schmelzenden Polymerkomponente zugeordnet.

Bekanntlich besitzen Polymere keinen scharf definierten Schmelzpunkt, sondern einen Schmelzbereich, wobei sich jedoch den kristallinen Bereichen eines Polymeren ein Kristallitschmelzpunkt zuordnen lässt. Dieser Kristallitschmelzpunkt liegt stets höher als der Schmelzpunkt oder Schmelzbereich der nicht-kristallinen Bestandteile. Der schmelzeflüssige Zustand ist dadurch beschrieben, dass der Schubmodul gegen Null geht. Im Falle von Polymeren mit kristallinen Bereichen sind letztere dann nicht mehr nachweisbar. Der Schubmodul kann beispielsweise nach ISO 6721-1 & 2 bestimmt werden. Bei der vorliegenden Erfindung wird jede Ausgangsfolienbahn auf eine Temperatur erwärmt, bei der für die niedrig schmelzende Polymerkomponente der Schubmodul Null beträgt und für die hoch schmelzende Polymerkomponente der Schubmodul nicht Null ist. Bei der niedrig schmelzenden Polymerkomponente sind dann keine kristallinen Bereiche mehr nachweisbar und die niedrig schmelzende Polymerkomponente befindet sich im schmelzeflüssigen Zustand. Hingegen sind bei der hoch schmelzenden Polymerkomponente noch kristalline Bereiche nachweisbar und diese befindet sich unterhalb des schmelzeflüssigen Zustands. In Summe ist der Schubmodul des gesamten Polymermaterials der Ausgangsfolienbahn somit nicht Null und kristalline Bereiche der hoch schmelzenden Polymerkomponente sind noch nachweisbar. Es liegt also eine teilweise aufgeschmolzene Folienbahn vor.

Als Materialien für die beiden Polymerkomponenten der Ausgangsfolienbahnen kommen im Prinzip alle thermoplastischen Polymere in Betracht, die über entsprechende Schmelzpunkte verfügen. Hierzu sind zahlreiche Handelsprodukte auf dem Markt erhältlich. Vorzugsweise werden verschiedene Polyolefine, insbesondere Polyethylene, Polypropylene, Copolymerisate von Ethylen und Propylen, Copolymerisate von Ethylen und Propylen mit anderen Comonomeren, oder Gemische davon eingesetzt. Weiterhin sind Ethylenvinylacetat (EVA), Ethylenacrylat (EA), Ethylenethylacrylat (EEA), Ethylenacrylsäure (EAA), Ethylenmethylacrylat (EMA), Ethylenbutylacrylat (EBA), Polyester (PET), Polyamid (PA), z.B. Nylon, Ethylenvinylalkohole (EVOH), Polystyrol (PS), Polyurethan (PU), thermoplastische Olefinelastomere oder thermoplastische Ether-Ester-Blockelastomere (TPE-E) geeignet.

Die Gesamtmenge an niedrig schmelzender Polymerkomponente beträgt vorzugsweise 90 bis 10 Gew.-%, insbesondere 90 bis 20 Gew.-%, bevorzugt 80 bis 30 Gew.-%, stärker bevorzugt 80 bis 40 Gew.-%, am meisten bevorzugt 70 bis 50 Gew.-%. Die Gesamtmenge an hoch schmelzender Polymerkomponente beträgt vorzugsweise 10 bis 90 Gew.-%, insbesondere 10 bis 80 Gew.-%, bevorzugt 20 bis 70 Gew.-%, stärker bevorzugt 20 bis 60 Gew.-%, am meisten bevorzugt 30 bis 50 Gew.-%, jeweils bezogen auf 100 Gew.-% niedrig schmelzende und hoch schmelzende Polymerkomponente. Alternativ beträgt vorzugsweise die Gesamtmenge an niedrig schmelzender Polymerkomponente 85 bis 15 Gew.-%, insbesondere 75 bis 25 Gew.-%, und die Gesamtmenge an hoch schmelzender Polymerkomponente 15 bis 85 Gew.-%, insbesondere 25 bis 75 Gew.-%, wiederum bezogen auf 100 Gew.-% niedrig und hoch schmelzende Komponente. Bei diesen Mengenangaben kann es sich beispielsweise bei der niedrig schmelzenden Polymerkomponente um ein oder mehrere Polyethylen(e) und bei der hoch schmelzenden Polymerkomponente um ein oder mehrere Polypropylen(e) handeln.

In einer besonders bevorzugten Ausführungsform enthält jede Ausgangsfolienbahn wenigstens ein Polyethylen als niedrig schmelzende Polymerkomponente und wenigstens ein Polypropylen als hoch schmelzende Polymerkomponente.

Vorzugsweise enthält die niedrig schmelzende Polymerkomponente Ethylenpolymere oder besteht aus diesen, wobei sowohl Ethylenhomopolymere als auch Ethylencopolymere mit Ethylen als Hauptmonomerem sowie Gemische (Blends) von Ethylenhomopolymeren und Ethylencopolymeren geeignet sind. Geeignete Ethylenhomopolymere sind LDPE (Low Density Polyethylene), LLDPE (Linear Low Density Polyethylene), MDPE (Medium Density Polyethylene) und HDPE (High Density Polyethylene). Bevorzugte Comonomere für Ethylencopolymere sind andere Olefine als Ethylen mit Ausnahme von Propylen, z. B. Buten, Hexen oder Octen. Vorzugsweise liegt bei den Ethylencopolymeren der Comonomergehalt unter 20 Gew.-%, insbesondere unter 15 Gew.-%. In einer bevorzugten Ausführungsform besteht die niedrig schmelzende Polymerkomponente ausschließlich aus einem Ethylenhomopolymer oder Gemischen von Ethylenhomopolymeren, z. B. aus LDPE und LLDPE, die jeweils in Mengen von 10 bis 90 Gew.-% enthalten sein können, sowie 0 bis 50 Gew.-% MDPE. Spezielle Beispiele sind ein Polyethylen aus 60 Gew.-% LDPE und 40 Gew.-% LLDPE oder ein Polyethylen aus 80 Gew.-% LDPE und 20 Gew.-% LLDPE.

Neben den Ethylenhomopolymeren und/oder Ethylencopolymeren kann die niedrig schmelzende Polymerkomponente auch andere thermoplastische Polymere enthalten. Diese thermoplastischen Polymeren sind nicht beschränkt, solange hierdurch die Temperatur, bei der sich die gesamte niedrig schmelzende Polymerkomponente im schmelzeflüssigen Zustand befindet, nicht zu nahe an die Temperatur heranrückt, bei der sich die hoch schmelzende Polymerkomponente im schmelzeflüssigen Zustand befindet. Es ist auch möglich, dass die niedrig schmelzende Polymerkomponente ein Polypropylen enthält, dessen Schmelzpunkt oder Schmelzbereich nicht höher als der eines Ethylenhomopolymeren oder Ethylencopolymeren liegt oder aber zwar höher als diese, jedoch immer noch tiefer als die eingesetzte Erwärmungstemperatur. Bekanntlich gibt es hochkristallines isotaktisches, weniger kristallines syndiotaktisches und amorphes ataktisches Polypropylen, die über unterschiedliche Schmelzpunkte, Schmelzbereiche oder Kristallitschmelzpunkte verfügen. Bei Verwendung von amorphem ataktischem Polypropylen, das einen wesentlich niedrigeren Schmelzpunkt oder Schmelzbereich als isotaktisches und gegebenenfalls auch syndiotaktisches Polypropylen hat, wäre dieses in Abhängigkeit von der Erwärmungstemperatur gegebenenfalls der niedrig schmelzenden Polymerkomponente zuzurechnen.

Vorzugsweise enthält die hoch schmelzende Polymerkomponente wenigstens ein Polypropylen, dessen Schmelzpunkt, Schmelzbereich oder Kristallitschmelzpunkt wesentlich höher als der der niedrig schmelzenden Polymerkomponente liegt. Geeignetes Polypropylen ist insbesondere isotaktisches Polypropylen. Es kann auch syndiotaktisches Polypropylen verwendet werden, sofern dessen Schmelzpunkt, Schmelzbereich oder Kristallitschmelzpunkt wesentlich höher als der der niedrig schmelzenden Polymerkomponente liegt. Geeignete Polypropylene sind im Handel erhältlich, beispielsweise für die Herstellung von Blasfolien und/oder Cast-Folien (Gießfolien).

Die hoch schmelzende Polymerkomponente kann sowohl Propylenhomopolymere als auch Propylencopolymere mit Propylen als Hauptmonomerem umfassen. Bei den Propylencopolymeren ist hierbei der Anteil des Comonomeren, d. h. das Nicht-Propylen, in Anhängigkeit von den anderen Komponenten und der Erwärmungstemperatur der niedrig oder hoch schmelzenden Polymerkomponente zuzurechnen. Geeignete Comonomere für Propylencopolymere sind andere Olefine als Propylen, vorzugsweise Ethylen. Bei den Propylen-Ethylen-Copolymeren beträgt der Ethylenanteil vorzugsweise 2 bis 30 Gew.-%, besonders bevorzugt 2 bis 20 Gew.-% und insbesondere 2 bis 15 Gew.-%, wobei in der Praxis bei einem Ethylengehalt von 3 bis 20 Gew.-% sehr gute Ergebnisse erhalten werden. Diese Zahlenwerte gelten auch für die anderen Olefine.

Nachfolgend sind für einige Polyethylene und Polypropylene die Schmelzbereiche angegeben:
LDPE: 110 - 114°C;
LLDPE: 115 - 130°C;
HDPE: 125 - 135 °C;
Propylen-Homopolymere: 150 - 165°C;
Propylen-Ethylen-Copolymere: 120 - 162°C, bei sehr wenig Ethylen auch höhere Temperaturen möglich;
Bimodale Propylen-Ethylen-(Homo)Copolymere: 110 - 165 °C.

Es ist auch möglich, sogenannte bimodale Polypropylene zu verwenden. Dabei handelt es sich um zwei verschiedene Polypropylene mit jeweils unterschiedlichem Copolymeranteil, die in einem Rohstoff zusammengefasst sind. Ein solches bimodales Polypropylen hat zwei Kristallitschmelzpunkte, wobei in der Regel über eine DSC-Analyse auch die ungefähren Anteile der zwei Polypropylene bestimmt werden können. Als Beispiel sei ein bimodales Polypropylen mit den Kristallitschmelzpunkten 125 °C und 143 °C mit einem Anteil der zwei verschiedenen Polypropylene von 25/75 genannt. Bei einer Erwärmungstemperatur von 130 °C wären erfindungsgemäß die 25 % Polypropylen mit Kristallitschmelzpunkt 125 °C der niedrig schmelzenden Polymerkomponente und die 75 % Polypropylen mit Kristallitschmelzpunkt 143 °C der hoch schmelzenden Polymerkomponente zuzurechnen.

In einer besonderen Ausführungsform wird eine Ausgangsfolienbahn mit folgenden Polymerbestandteilen verwendet: 25 bis 80 Gew.-%, insbesondere 25 bis 60 Gew.-% eines LLDPE, z. B. ein Ethylen-Octen-Copolymer mit 5 bis 15 Gew.-% Octenanteil; 20 bis 30 Gew.-% eines Propylen-Ethylen-Copolymeren mit 3 bis 12 Gew.-% Ethylen; und Rest LDPE; jeweils bezogen auf 100 Gew.-% niedrig und hoch schmelzende Polymerkomponente.

Ebenso wie sich in der niedrig schmelzenden Polymerkomponente spezielles geschmolzenes Polypropylen befinden kann, kann sich auch in der hoch schmelzenden Polymerkomponente ein spezielles nicht geschmolzenes Polyethylen befinden, das dann der hoch schmelzenden Polymerkomponente zugerechnet wird. Dies wird an folgendem Beispiel veranschaulicht. Eine für eine Ausgangsfolienbahn geeignete Rezeptur umfasst als Polymerbestandteile: 30 Gew.-% LDPE (Schmelzpunkt 112 °C), 30 Gew.-% LLDPE (Schmelzpunkt 124 °C), 20 Gew.-% HDPE (Schmelzpunkt 130 °C) und 20 Gew.-% Polypropylen (Schmelzpunkt 160 °C). Wird die Folienbahn auf eine Temperatur von 126 °C erwärmt, so befinden sich das LDPE und LLDPE erfindungsgemäß im schmelzeflüssigen Zustand, aber nicht nur das Polypropylen sondern auch das HDPE befinden sich nicht im schmelzeflüssigen Zustand.

Das erfindungsgemäße Verfahren kann auch mit gefüllten oder mikroporösen Ausgangsfolienbahnen durchgeführt werden.

Die Ausgangsfolienbahnen für die Durchführung des Verfahrens der Erfindung können durch beliebige, im Stand der Technik bekannte Verfahren hergestellt werden. Beispielsweise kann die Ausgangsfolienbahn hergestellt werden, indem die Polymerbestandteile, und gegebenenfalls Füllstoffe, in einem Extruder, z.B. einem Compounding-Extruder, auf eine Temperatur deutlich über der Schmelzflusstemperatur der Polymerbestandteile (z.B. über 200°C) erhitzt und verschmolzen werden. Dem schließt sich ein Cast-Verfahren (Gießverfahren), z.B. über eine Schlitzdüse, oder ein Blasverfahren an. Diese Verfahren sind im Stand der Technik bekannt. Beim Schlitzdüsenverfahren wird eine Folie durch eine Breitschlitzdüse extrudiert. Bevorzugt ist das Blasverfahren, bei dem ein Blasschlauch gebildet wird. Die gebildete Schlauchfolie kann flach aufeinandergelegt und an den Enden aufgeschlitzt oder aufgetrennt werden, so dass zwei Folienbahnen entstehen, von denen jede als Ausgangsfolienbahn verwendet werden kann. Der Vorteil des Aufschlitzens oder Auftrennens des Schlauches liegt darin, dass Luft entweichen kann. Alternativ kann der flachgelegte Schlauch ohne Aufschlitzen oder Auftrennen in Form von zwei Ausgangsfolienbahnen im Verfahren der Erfindung eingesetzt werden.

In bevorzugten Ausführungsformen wird wenigstens eine Ausgangsfolienbahn oder jede Ausgangsfolienbahn in Maschinenrichtung (MD), Querrichtung (CD), oder in Maschinen- und Querrichtung gereckt. Wird eine mikroporöse Ausgangsfolienbahn verwendet, kann zur Erzeugung der Mikroporosität die extrudierte Folie einem Reckvorgang unterzogen werden. Daneben ist auch ein Ringrolling möglich.

In bevorzugten Ausführungsformen ist wenigstens eine Ausgangsfolienbahn oder jede Ausgangsfolienbahn gereckt. Ein Recken, Strecken oder Verstrecken einer Folie bedeutet eine Dehnung der Folie in eine angegebene Richtung, was zu einer Verringerung der Foliendicke führt. Die Folie kann in Maschinen- oder Längsrichtung (MD) gereckt sein, beispielsweise durch ein Reckwerk, das zwei oder mehrere Walzen umfasst, z.B. drei Walzen, die bei unterschiedlicher Geschwindigkeit angetrieben werden. Die Folie kann beispielsweise bei einem Reckverhältnis von 1:1,5 gereckt werden, was bedeutet, dass die Foliendicke von z.B. 15 µm auf 10 µm verringert wird. Es ist auch möglich, die Folienbahn zusätzlich einer Querverstreckung (CD) zu unterwerfen. Ein solches biaxiales Verstrecken kann beispielsweise durch auf dem Markt erhältliche Reckmaschinen, z.B. von der Firma Brückner, erreicht werden. Das verwendete Reckverhältnis hängt von der Folienrezeptur und den gewählten Verfahrensparametern ab und kann wenigstens 1:1,2, bevorzugt wenigstens 1:1,5, insbesondere wenigstens 1:2, stärker bevorzugt wenigstens 1:2,5, mehr bevorzugt wenigstens 1:3 oder wenigstens 1:4 betragen.

In bevorzugten Ausführungsformen enthält wenigstens eine Ausgangsfolienbahn Füllstoffe. In beispielhaften Ausführungsformen enthalten zwei Ausgangsfolienbahnen Füllstoffe. Geeignete Füllstoffe unterliegen keinen Beschränkungen und sind dem Fachmann bekannt. Es eignen sich alle Stoffe, die auf eine bestimmte Größe gemahlen werden können, im Extruder nicht schmelzen und nicht gereckt werden können. Besonders geeignet sind anorganische Füllstoffe, wie Kreide (Calciumcarbonat), Ton, Kaolin, Calciumsulfat (Gips) oder Magnesiumoxid. Daneben eignen sich auch synthetische Füllstoffe, wie Kohlenstofffasern, Cellulosederivate, gemahlene Kunststoffe oder Elastomere. Am meisten bevorzugt ist Calciumcarbonat oder Kreide wegen seines günstigen Preises, aber auch unter dem Gesichtspunkt der Nachhaltigkeit. Der Füllstoff kann eine Partikelgröße von z.B. 0,8 bis 2 µm haben. Falls ein Füllstoff mit einer gleichmäßigeren Partikelgröße als bei Kreide gewünscht ist, besteht auch die Möglichkeit, synthetische Füllstoffe mit einheitlicher Partikelgröße oder Partikelgrößenverteilung zu verwenden. Die Folie kann wenig Füllstoffe enthalten, z. B. 5 bis 45 Gew.-% oder 10 bis 50 Gew.-%, so dass sich bei einem Reckvorgang zwar Poren ausbilden, diese aber isoliert vorliegen und die Folie nicht atmungsaktiv ist. Um eine Atmungsaktivität der Folie zu erreichen, werden zweckmäßigerweise wenigstens 35 Gew.-% Füllstoff, insbesondere wenigstens 45 Gew.-% Füllstoff, bevorzugt wenigstens 55 Gew.-% Füllstoff, stärker bevorzugt wenigstens 65 Gew.-% Füllstoff, bezogen auf 100 Gew.-% der gesamten Rezeptur der Ausgangsfolienbahn, einschließlich Füllstoff(en), verwendet. Die Obergrenze an Füllstoff wird dadurch bestimmt, dass keine Poren mehr, sondern Löcher entstehen, oder die Folie reißt. Geeignete Folienrezepturen mit Füllstoff können vom Fachmann routinemäßig bestimmt werden. Besonders geeignet ist eine Rezeptur mit 35 bis 75 Gew.-%, insbesondere 45 bis 75 Gew.-%, bevorzugt 55 bis 70 Gew.-%, Füllstoff bezogen auf 100 Gew.-% Ausgangsfolienbahn. Beispielhafte Rezepturen für nicht-atmungsaktive Folien umfassen 5 bis 50 Gew.-%, insbesondere 10 bis 40 Gew.-%, Füllstoffe, bezogen auf 100 Gew.-% Ausgangsfolienbahn. Beispielhafte Rezepturen für atmungsaktive Folien umfassen 35 bis 80 Gew.-%, insbesondere 45 bis 75 Gew.-%, Füllstoffe, bezogen auf 100 Gew.-% Ausgangsfolienbahn. Es ist darauf zu achten, den Anteil der niedrig schmelzenden Komponente nicht so hoch zu wählen, dass eine Atmungsaktivität zwar erreicht wird, diese aber wieder verloren geht, weil die Poren sich wieder verschließen.

Bei Verwendung einer mikroporösen Ausgangsfolienbahn hat diese vorzugsweise Mikroporen in der Größe von Größe von 0,1 bis 5 µm, insbesondere von 0,1 bis 3 µm oder von 0,2 bis 1 µm. Daneben können auch einige wenige größere Poren vorliegen.

Jede Ausgangsfolienbahn kann einschichtig oder mehrschichtig sein, also monoextrudiert bzw. coextrudiert sein. Es gibt keine Beschränkung bezüglich der Anzahl der verwendeten Schichten oder Lagen. Es können eine oder mehrere Schichten oder Lagen vorliegen, z. B. eine Schicht, zwei Schichten, drei Schichten oder vier Schichten. Möglich sind auch z. B. bis 5, 7 oder 9 Schichten. Die Schichten oder Lagen können gleiche oder unterschiedliche Rezepturen aufweisen, wobei die Zuordnung zur niedrig oder hoch schmelzenden Polymerkomponente jeweils durch den Kristallitschmelzpunkt bestimmt wird. Die Schichten oder Lagen einer Ausgangsfolienbahn können durch Coextrusion hergestellt werden. Es besteht keine Beschränkung bei der Anzahl der coextrudierten Schichten oder Lagen einer Ausgangsfolienbahn. In anderen Ausführungsformen ist wenigstens eine Ausgangsfolienbahn oder jede Ausgangsfolienbahn nicht coextrudiert.

Die Ausgangsfolienbahnen können durch Blasextrusion oder Cast-Extrusion oder eine Kombination davon hergestellt werden. Beispielsweise kann wenigstens eine Ausgangsfolienbahn durch Blasextrusion und wenigstens eine andere Ausgangsfolienbahn durch Cast-Extrusion hergestellt werden. Es besteht keine Beschränkung bei der Kombination von blasextrudierten und/oder cast-extrudierten Ausgangsfolienbahn.

Die Ausgangsfolienbahnen können in beispielhaften Ausführungsformen wie nachstehend beschrieben hergestellt werden:
- blasextrudiert;
- breitschlitz- bzw. cast-extrudiert (mono- oder coextrudiert);
- mono- oder coextrudiert;
- blasextrudiert, geschlitzt, auf zwei separate Bahnen bzw. separate Rollen;
- blasextrudiert, geschlitzt, auf zwei oder mehrere separate Bahnen gleichzeitig;
- blasextrudiert, geschlitzt, flachgelegt als nicht getrennter Schlauch;
- blasextrudiert, geschlitzt in zwei oder mehrere separate Bahnen, die von verschiedenen Extrudern kommen;
- cast-extrudiert in zwei oder mehrere separate Bahnen gleichzeitig.

Es besteht keine Beschränkung in der Anzahl der Ausgangsfolienbahnen. Es besteht keine Beschränkung in der Kombination aus blas- oder cast-extrudierten Ausgangsfolienbahnen. Ebenso besteht keine Beschränkung bei der Anzahl der coextrudierten Schichten in der Kombination aus blas- oder cast-extrudierten Ausgangsfolienbahnen.

Es ist auch möglich, die Ausgagnsfolienbahnen Inline herzustellen. In diesem Fall liegt ein Produktionsschritt für die Prozesse Extrusion und Recken (MDO, biaxial oder Ringrolling) sowie die weitere Verarbeitung (z. B. Prägen und Bedruckung) vor.

Die beim Verfahren der Erfindung eingesetzten Ausgangsfolienbahnen können eingefärbt sein, z. B. weiß mit Titandioxid. Weiterhin können die Ausgangsfolienbahnen übliche Zusatzstoffe und Verarbeitungshilfsmittel enthalten. Insbesondere handelt es sich hierbei neben den bereits genannten Füllstoffen um Pigmente oder andere farbgebende Stoffe, Antihaftmittel, Gleitmittel, Verarbeitungshilfsmittel, antistatische Mittel, keimverhindernde Mittel (Biozide), Antioxidationsmittel, Wärmestabilisierungsmittel, Stabilisierungsmittel gegenüber UV-Licht oder andere Mittel zur Eigenschaftsmodifizierung. Typischerweise werden solche Stoffe, ebenso wie Füllstoffe, bereits vor dem erfindungsgemäßen Erwärmen der Ausgangsfolienbahn zugegeben, z.B. bei deren Herstellung in die Polymerschmelze oder vor dem Extrudieren zu einer Folie.

Die Ausgangsfolienbahnen weisen bevorzugt Flächengewichte im Bereich unter 50 g/m², insbesondere unter 40 g/m², bevorzugt unter 30 g/m², stärker bevorzugt unter 20 g/m², auf. Möglich sind auch Flächengewichte im Bereich unter 10 g/m² oder unter 5 g/m². Bevorzugte Flächengewichte liegen im Bereich von 1 bis 30 g/m², 1 bis 25 g/m² oder 1 bis 20 g/m², insbesondere von 1 bis 15 g/m², stärker bevorzugt von 2 bis 10 g/m² oder 7 bis 20 g/m². Die Flächengewichte können auch 1 bis 10 g/m², 5 bis 10 g/m² oder 5 bis 15 g/m² betragen. Die Ausgangsfolienbahnen können Dicken im Bereich von 2 bis 30 µm, insbesondere von 2 bis 15 µm, 5 bis 20 µm oder 5 bis 10 µm aufweisen.

Die Ausgangsfolienbahnen werden erfindungsgemäß gemeinsam über wenigstens einen Heizzylinder erwärmt und anschließend durch einen gekühlten Walzenspalt geführt. Vorzugsweise werden zwei Ausgangsfolienbahnen erwärmt. Die beiden Ausgangsfolienbahnen können dem Heizzylinder getrennt oder gemeinsam zugeführt werden. Getrennte Ausgangsfolienbahnen können zum Beispiel von getrennten Rollen kommen. Eine gemeinsame Zuführung ergibt sich zum Beispiel, wenn ein Blasschlauch flachgelegt und nicht aufgeschlitzt oder an den beiden flachgelegten Folienkanten in Maschinenrichtung aufgeschlitzt wird, so dass der flachgelegte Blasschlauch, der zwei Ausgangsfolienbahnen darstellt, von einer Rolle kommt.

Beim erfindungsgemäßen Verfahren wird eine Ausgangsfolienbahn zusammen mit wenigstens einer weiteren Ausgangsfolienbahn, vorzugsweise einer weiteren Ausgangsfolienbahn, dem Heizzylinder zugeführt. Es kommt nicht darauf an, welche der Ausgangsfolienbahnen auf dem Heizzylinder aufliegt.

Bei dem Verfahren der Erfindung wird die Erwärmung jeder Ausgangsfolienbahn bis zum oder oberhalb dem schmelzeflüssigen Zustand der niedrig schmelzenden Polymerkomponente und unterhalb des schmelzeflüssigen Zustands der hoch schmelzenden Polymerkomponente durchgeführt. Bis zum schmelzeflüssigen Zustand bedeutet hierbei, dass sich die niedrig schmelzende Polymerkomponente im schmelzeflüssigen Zustand befindet. Es wird jedoch nur so hoch erwärmt, dass sich die hoch schmelzende Polymerkomponente nicht im schmelzeflüssigen Zustand befindet.

Um eine stabile Prozessführung auch über einen längeren Zeitraum zu ermöglichen, sollten zweckmäßigerweise die (Kristallit-)Schmelzpunkte der niedrig und hoch schmelzenden Polymerkomponenten nicht zu nahe beieinanderliegen. Vorzugsweise liegt der Kristallitschmelzpunkt der niedrig schmelzenden Polymerkomponente oder, bei Anwesenheit mehrerer niedrig schmelzender Polymerkomponenten, der Kristallitschmelzpunkt derjenigen mit dem höchsten Kristallitschmelzpunkt davon, mindestens etwa 5 °C, verzugsweise mindestens etwa 10 °C und insbesondere mindestens etwa 20 °C unterhalb des Kristallitschmelzpunkts oder des schmelzeflüssigen Zustandes der hoch schmelzenden Polymerkomponente oder, bei Anwesenheit mehrerer hoch schmelzender Polymerkomponenten, derjenigen mit dem niedrigsten Kristallitschmelzpunkt davon.

Zur Erreichung des schmelzeflüssigen Zustands der niedrig schmelzenden Polymerkomponente der Ausgangsfolienbahnen, jedoch nicht des schmelzeflüssigen Zustands der hoch schmelzenden Polymerkomponente der Ausgangsfolienbahnen, unterliegt der speziell gewählte Temperaturabstand keinen besonderen Beschränkungen, sofern die vorgenannte Bedingung erfüllt ist. Der gewählte Temperaturabstand wird zweckmäßigerweise durch praktische Überlegungen bezüglich Sicherheit der Verfahrensdurchführung oder durch wirtschaftliche Überlegungen bestimmt. Wenn z.B. bei einer bestimmten Temperatur die niedrig schmelzende Polymerkomponente jeder Ausgangsfolienbahn aufgeschmolzen ist, bringt eine weitere Temperaturerhöhung keine besseren Ergebnisse. Zudem steigt der Wärmeverbrauch und man kommt gegebenenfalls zu nahe an den Schmelzbereich der hoch schmelzenden Polymerkomponente einer Ausgangsfolienbahn heran, so dass das Verfahren schwieriger durchzuführen ist. Vorzugsweise wird das Verfahren der Erfindung so durchgeführt, dass die Erwärmung der Ausgangsfolienbahn auf 5 bis 20 °C, bevorzugt 5 bis 15 °C oder 10 bis 20°C, insbesondere 10 bis 15°C oder 15 bis 20 °C, unterhalb des Kristallitschmelzpunkts der hoch schmelzenden Polymerkomponente der Ausgangsfolienbahnen erfolgt. Alternativ wird die Erwärmung insbesondere bei einer Temperatur im Bereich von 1 bis 20 °C, bevorzugt 2 bis 10°C, oberhalb des Kristallitschmelzpunkts oder schmelzeflüssigen Zustands der niedrig schmelzenden Polymerkomponente(n) durchgeführt. Es ist sicherzustellen, dass die Kristallitschmelzpunkte der niedrig schmelzenden Polymerkomponente(n) erreicht werden.

Erfindungsgemäß kann die Erwärmung der wenigstens zwei Ausgangsfolienbahnen mittels wenigstens einer Heizwalze erfolgen. Vorzugsweise erfolgt die Erwärmung mittels einer oder mehrerer Heizwalzen, bei denen es sich um Kontaktwalzen handelt, die mittels eines Wärmeträgers, z.B. Dampf, Wasser, Öl, auf die vorgegebene Temperatur erhitzt werden. In einer bevorzugten Ausführungsform wird eine einzelne Heiz- oder Kontaktwalze eingesetzt. Es ist aber auch möglich, zwei oder mehrere Heizwalzen einzusetzen, wobei sichergestellt werden muss, dass der schmelzeflüssige Zustand der niedrig schmelzenden Polymerkomponente jeder Ausgangsfolienbahn vor dem Kühlwalzenspalt erreicht wird. Um zu gewährleisten, dass die Ausgangsfolienbahnen die Temperatur der Heizwalze tatsächlich erreichen oder dass bei hohen Produktionsgeschwindigkeiten (bei denen die Oberflächentemperatur des Heizzylinders höher im Vergleich zu den Folien ist) der schmelzeflüssige Zustand der niedrig schmelzenden Polymerkomponente sicher erreicht wird, ist für eine ausreichende Verweilzeit der Ausgangsfolienbahn auf der Heizwalzenoberfläche zu sorgen. Dies kann über eine entsprechende Umschlingungsstrecke des Heizzylinders, den Heizwalzendurchmesser und/oder die Folienbahngeschwindigkeit nach Maßgabe der Foliendicke erfolgen. Zweckmäßigerweise kann eine Heizwalze mit antihaft-beschichteter Oberfläche verwendet werden, um ein leichteres Ablösen der auf der Heizwalze aufliegenden Folienbahn zu erreichen und damit ein Abreißen der Folienbahn zu verhindern. Dadurch wird eine Verschiebung des Ablösepunktes in Drehrichtung der Heizwalze vermieden und es ist keine oder nur mehr eine geringe Voreilung nötig. Beispielsweise wird hierzu eine PTFE (Polytetrafluorethylen)-beschichtete Heizwalze verwendet.

Die Erwärmung der Folienbahnen kann mit anderen Erwärmungsmethoden, wie Strahlungswärme, z.B. mit Infrarotheizung oder -strahlern, unterstützt werden. Es kann zusätzlich zu einer oder mehreren Heizwalzen eine andere Erwärmung, z.B. Infrarotheizung, vorgesehen sein.

Erfindungsgemäß wird die mehrlagige Folienbahn nach der Erwärmung durch einen gekühlten Walzenspalt geführt. Die den Kühlwalzenspalt bildenden Walzen sind so gekühlt, dass eine schnelle und schlagartige Abkühlung erreicht wird. Eine Abkühlung auf eine Temperatur unterhalb des Kristallitschmelzpunktes der niedrig schmelzenden Polymerkomponente wenigstens einer Ausgangsfolienbahn, bevorzugt von jeder Ausgangsfolienbahn, vorzugsweise auf wenigstens 5 °C darunter, insbesondere auf wenigstens 10 °C darunter, ist zweckmäßig. Bevorzugte Abkühlbereiche sind 5 bis 10 °C, stärker bevorzugt 10 bis 30 °C, unterhalb des Kristallitschmelzpunktes der niedrig schmelzenden Polymerkomponente einer Ausgangsfolienbahn oder jeder Ausgangsfolienbahn. Beispielsweise kann die Kühlung der Walzen mit Wasser in einem Temperaturbereich von 5 bis 20 °C, z.B. Wasser von etwa 10 °C, erfolgen. Der Abstand zwischen der Heizwalze oder, bei Verwendung mehrerer Heizwalzen, der letzten Heizwalze und/oder anderer Erwärmungsquellen und dem Kühlwalzenspalt wird hierbei wegen möglicher Wärmeverluste nicht zu groß gewählt.

Bei dem Kühlwalzenspalt kann es sich im einfachsten Fall z.B. um einen Glattwalzenspalt mit zwei glatten Walzen handeln. Im Fall von Hygienefolien wird der Walzenspalt vorzugsweise von einem Walzenpaar mit einer Strukturwalze und einer Glattwalze (z. B. Gummiwalze) gebildet, wodurch die Folienbahn eine strukturierte Oberfläche erhält. Bevorzugte Strukturen im Hygienebereich sind Mikrostrukturen, z.B. ein Pyramidenstumpf. Vorzugsweise besteht der gekühlte Walzenspalt aus einer Stahlwalze und einer im Gegendruck arbeitenden Gummiwalze, wobei die Stahlwalze mit der strukturierten Oberfläche versehen ist. Die Stahlwalze kann mit einer textilähnlichen Gravur ausgestattet sein, die das textile Erscheinungsbild der Oberfläche der Folie verstärkt. Eine Prägestruktur der Stahlwalze reduziert weiterhin des Glanzgrad der Folie.

Die Geschwindigkeit der den Kühlwalzenspalt bildenden Walzen kann so gewählt werden, dass diese gleich der Geschwindigkeit der Heizwalze oder, bei Verwendung mehrerer Heizwalzen, der letzten Heizwalze ist, so dass die Folie dazwischen nicht gereckt wird. Die Geschwindigkeit der den Kühlwalzenspalt bildenden Walzen kann auch so gewählt werden, dass diese größer oder kleiner als die Geschwindigkeit der Heizwalze oder, bei Verwendung mehrerer Heizwalzen, der letzten Heizwalze ist, so dass die Folie dazwischen gereckt oder geschrumpft wird. Wegen Wärmeverlusten sollte der Abstand zwischen Heizwalze und Kühlwalzenspalt möglichst geringgehalten werden.

In bevorzugten Ausführungsformen wird die mehrlagige Folienbahn zwischen Heizzylinder und Kühlwalzenspalt gereckt. Wesentlich ist, dass sich die Folienbahn während dieses Reckvorgangs im teilweise geschmolzenen Zustand befindet. Das Reckverhältnis hängt von der Folienrezeptur und den gewählten Verfahrensparametern ab und beträgt vorzugsweise wenigstens 1:1,2, bevorzugt wenigstens 1:1,5, insbesondere wenigstens 1:2, stärker bevorzugt wenigstens 1:2,5, mehr bevorzugt wenigstens 1:3 oder wenigstens 1:4.

In einer bevorzugten Ausführungsform der Erfindung wird das Recken dadurch bewirkt, dass die den gekühlten Walzenspalt bildenden Kühlwalzen bei einer höheren Geschwindigkeit als die Heizwalze angetrieben werden. In einer anderen bevorzugten Ausführungsform der Erfindung sind vor dem Kühlwalzenspalt zwei oder mehr Walzen vorhanden, von denen wenigstens zwei mit unterschiedlicher Geschwindigkeit betrieben werden, so dass die Folienbahn zwischen diesen beiden Walzen gereckt wird, und wobei wenigstens die erste der zwei oder mehreren Walzen als Heizwalze ausgebildet ist. Es ist auch möglich, dass die zweite und die gegebenenfalls weiteren Walzen ebenfalls als Heizwalze ausgebildet sind. Insbesondere bei Vorhandensein mehrerer Walzen ist aber auch möglich, dass eine der Walzen als Kühlwalze ausgebildet ist. Eine Kühlwalze bewirkt eine einseitige Abkühlung der Folienbahn und führt damit zu einer langsamen Abkühlung der Folie. Im Gegensatz dazu bewirkt der erfindungsgemäß vorgesehene Kühlwalzenspalt aufgrund der zwei Kühlwalzen eine zweiseitige Kühlung der Folienbahn, was eine schnelle Kühlung herbeiführt. Wenn eine Kühlwalze eingesetzt wird, ist vor dem Kühlwalzenspalt nochmals eine Erwärmung der Folienbahn in den teilweise geschmolzenen Zustand erforderlich, was zweckmäßigerweise wieder über eine Heizwalze erfolgen kann. Möglich sind also beispielsweise die Anordnungen Heizwalze - Heizwalze - gekühlter Walzenspalt oder Heizwalze - Kühlwalze - Heizwalze - gekühlter Walzenspalt.

In Abhängigkeit von den Folienparametern und anderen Verfahrensbedingungen bewegen sich die Folienbahngeschwindigkeiten im Bereich von 50 bis 900 m/min. Die Geschwindigkeit der Heizwalze(n) beträgt vorzugsweise 50 bis 900 m/min, insbesondere 50 bis 800 m/min, bevorzugt 100 bis 600 m/min. Die Geschwindigkeit der den Kühlwalzenspalt bildenden Walzen beträgt vorzugsweise 50 bis 900 m/min, insbesondere 50 bis 800 m/min, bevorzugt 100 bis 600 m/min. Die Geschwindigkeiten der Heizwalze(n) und Kühlwalzen werden so gewählt, dass in Abhängigkeit von der Folienrezeptur und den gewählten Verfahrensparametern diese gleich sind oder aber verschieden sind, so dass die Folie im gewünschten Verhältnis gereckt oder geschrumpft (Annealing) wird.

Das erfindungsgemäße Verfahren ermöglicht die Herstellung von mehrlagigen Folien mit sehr geringen Flächengewichten von z. B. 2, 3, 4, 5, 6, 7, 8, 9, 10, 11, 12, 13, 14, 15, 16, 17, 18, 19, 20 oder 25 g/m². Die entsprechenden Foliendicken liegen im Bereich von z. B. 25, 20, 19, 18, 17, 16, 15, 14, 13, 12, 11, 10, 9, 8, 7, 6 oder auch nur 5 µm. Bevorzugte Folien weisen eine Dicke im Bereich von 2 bis 13 µm oder 4 bis 25 µm auf oder haben ein Flächengewicht von 1 bis 15 g/m² oder von 4 bis 25 g/m² oder von 7 bis 20 g/m².

Die erfindungsgemäß erhaltenen Folien verfügen, trotzdem sie sehr dünn und mikroporös sind, über ausgezeichnete mechanische Eigenschaften und über zusätzlich noch immer hohe Durchstoßfestigkeiten (d.h. Widerstand gegenüber Superabsorberkörnern z.B. in Windeln) und hohe Thermostabilitäten (d.h. Widerstand gegenüber Hotmelt-Klebern).

Erfindungsgemäß erhaltene mehrlagige Folien können in bekannter Weise weiterverarbeitet werden. Beispielsweise können Single-Backsheets oder Vlies-Folien-Laminate daraus hergestellt werden. Zur Herstellung von Vlies-Folien-Laminaten können die Folien mit Vliesen mittels Klebstoffen, bevorzugt inline, verklebt werden. Daneben können Vlies-Folien-Laminate auch durch dem Fachmann bekanntes Thermobonding hergestellt werden, bei dem das Material von einer erfindungsgemäß erhaltenen Folie und/oder von Vlies punktuell mittels zweier beheizter Walzen, meist einer Prägewalze (gravierte Stahlwalze) und einer glatten Stahlwalze als Gegenwalze, durch hohe Temperatur und Druck angeschmolzen wird und dadurch die Folie und das Vlies miteinander verbunden werden. Darüber hinaus können Vlies-Folien-Laminate wie oben beschrieben auch mittels Thermolaminierung hergestellt werden. Eine Thermolaminierung ist insbesondere bei sehr dünnen Folien, z.B. unter 10 g/m² oder z. B. 4 g/m² bevorzugt. Daneben können Vlies-Folien-Laminate auch mittels Ultraschall-Laminierung (z. B. mit Ultraschall Herrmann Technologie) hergestellt werden. Die hergestellten Vlies-Folien-Laminate können in an sich bekannter Weise weiterverarbeitet werden, wobei auch ein Recken in Maschinen- oder Querrichtung oder in beide Richtungen möglich ist. Ebenso können Single-Backsheets weiterverarbeitet werden.

Figur 1 zeigt eine bevorzugte Ausführungsform zur Durchführung des erfindungsgemäßen Verfahrens. Eine Ausgangsfolienbahn 2 wird über eine Umlenkwalze 3 und eine Ausgangsfolienbahn 1 über eine Umlenk- und Anpresswalze 4 auf einen Heizzylinder 5 geführt. Bei dem Heizzylinder 5 oder der Heizwalze 5 handelt es sich z.B. um eine antihaftend beschichtete Stahlwalze, die mittels Wärmezufuhr auf die gewünschte Oberflächentemperatur erhitzt wird. Dort werden beide Bahnen erfindungsgemäß in den teilweise schmelzeflüssigen Zustand erwärmt und verbinden sich zu einer mehrlagigen Folienbahn. Die Folienbahn läuft von der Heizwalze 5 in einen Kühlwalzenspalt, der von den Walzen 6 und 7 gebildet wird. Die Walze 6 ist vorzugsweise als Struktur- oder Prägewalze ausgebildet, wodurch die Folienbahn eine Prägestruktur oder strukturierte Oberfläche erhält. Die Walze 7 ist vorzugsweise eine Gummiwalze. Das Walzenpaar 6/7 ist vorzugsweise wassergekühlt, z.B. mit Wasser von etwa 10 °C. Die den Kühlspalt bildenden Walzen 6 und 7 werden so angetrieben, dass gegenüber der Bahngeschwindigkeit der Heizwalze 5 eine höhere, niedrigere oder gleiche Geschwindigkeit besteht. Im Kühlwalzenspalt wird die Folienbahn schockartig gekühlt und geprägt. Nach dem Walzenpaar 6/7 kann die Folie direkt abgenommen werden, oder über Umlenkwalzen 8 und 9, die auch gekühlt werden können, kann die Folienbahn beispielsweise einer Reckung mittels der Ringrollingwalzen 10 und 11 zugeführt werden. Die fertige Folienbahn kann dann in an sich bekannter Weise weiterverarbeitet werden.

Die Erfindung ermöglicht aufgrund der Herstellung von Folien geringer Dicken eine Einsparung an Rohstoffen und trägt somit zur Ressourcenschonung und Nachhaltigkeit bei. Sie leistet somit einen Beitrag zur Umweltschonung. Dies gilt für Folien auf dem Hygienesektor wie auch für andere Anwendungen, vor allem in den Anwendungen, in denen die Folien in starkem Maße als Bestandteile von Wegwerfprodukten verwendet werden.

Die erfindungsgemäß hergestellte Folie bietet im Hinblick auf die eingangs beschriebenen Probleme im Stand der Technik folgende Verbesserungen und Vorteile:
- Die Folie erlaubt eine hohe Temperaturbelastung, z. B. bei Hotmeltklebern.
- Die Folie zeigt fast keinen Nachschrumpf.
- Da die Folien kaum messbaren Nachschrumpf zeigen, können sie inline direkt nach dem Reckvorgang bedruckt werden, beispielweise mit einem zwischengeschalteten "Heißprägeverfahren".
- Die Folie zeigt bei großen thermischen Belastungen, wie beispielsweise beim Auftrag von Hotmelt-Klebersystemen, höhere Widerstandskräfte bzw. geringes Schrumpfverhalten bzw. geringere sog. Burn Through-Effekte, wodurch die Lochbildung verringert wird bzw. nicht mehr auftritt.

Die erfindungsgemäß erhaltenen Folien können in vielen Bereichen verwendet werden. Sie finden Verwendung im Hygiene- oder Medikalbereich, z.B. als Wäscheschutzfolie oder allgemein als flüssigkeitsundurchlässige Sperrschicht, insbesondere als Backsheets in Windeln, Damenbinden, Betteinlagen oder in ähnlichen Produkten. Weiterhin können die Folien in anderen technischen Gebieten Anwendung finden, wie im Bausektor als Baufolien, z. B. für Dachunterspannbahnen, Estrichabdeckungen oder Wandabdeckungen, oder als Automobilbereich als Autoschutzfolien.

Erfindungsgemäß erhaltene Folien können in bekannter Weise weiterverarbeitet werden, beispielsweise zu Vlies-Folien-Laminaten. Zur Herstellung solcher Laminate können sie mit Klebstoffen, bevorzugt inline, verklebt werden. Daneben können Vlies-Folien-Laminate auch durch dem Fachmann bekanntes Thermobonding hergestellt werden, bei dem das Material von einer erfindungsgemäß erhaltenen Folie und/oder von Vlies punktuell mittels zweier beheizter Walzen, meist einer Prägewalze (gravierte Stahlwalze) und einer glatten Stahlwalze als Gegenwalze, durch hohe Temperatur und Druck angeschmolzen wird und dadurch die Folie und das Vlies miteinander verbunden werden. Darüber hinaus können Vlies-Folien-Laminate auch mittels Thermolaminierung, beispielsweise wie in EP 1 784 306 B1 beschrieben, hergestellt werden. Eine Thermolaminierung ist insbesondere bei sehr dünnen Folien, z.B. 4 g/m² bevorzugt. Alternativ können Vlies-Folien-Laminate auch mittels Ultraschall-Laminierung (z. B. mit Ultraschall Herrmann Technologie) hergestellt werden. Die hergestellten Vlies-Folien-Laminate können in an sich bekannter Weise weiterverarbeitet werden.

Die erfindungsgemäße Verfahren ermöglicht durch das Aufeinanderlegen der Ausgangsfolienbahnen auf dem Heizzylinder eine starke Verringerung der Anzahl der Fehlstellen und Löcher. Gleichzeitig werden über den teilweise schmelzflüssigen Zustand die Ausgangsfolienbahnen miteinander verbunden und thermisch behandelt. Ein Recken der mehrlagigen Folienbahn, beispielsweise zwischen dem Heizzylinder und dem Kühlwalzenspalt, führt zu einer Reduzierung des Flächengewichts oder der Dicke der Folienbahn, so dass der eventuelle Nachteil eines höheren Flächengewichts, bedingt durch den Einsatz von wenigstens zwei Ausgangsfolienbahnen, wieder kompensiert werden kann. Insgesamt wird durch die Erfindung der Ausschuss bei dünnen Polymerfolien reduziert und damit ein wesentlicher Beitrag zur Ressourcenschonung und Nachhaltigkeit geleistet.

Die Erfindung wird anhand des nachfolgenden Beispiels näher erläutert, ohne die Erfindung zu beschränken.

### BEISPIEL

Die Ausgangsfolienbahnen wurden durch übliche Blasextrusion bei einer Extrudertemperatur von 240 °C mit einer Rezeptur gemäß der Tabelle I hergestellt.

**TABELLE I**

| Menge Gew.-Teile | Bestandteil | Dichte, g/cm³ | Kristallitschmelzpunkt, °C |
|---|---|---|---|
| 59 | LDPE | 0,922-0,924 | 113 |
| 41 | LLDPE-Octen | 0,930 | 124 |
| 30 | Polypropylen¹⁾ | 0,90 | 162 |
| 5 | TiO₂-Weiß-konzentrat | 1,69 | - |

| | | | |
|---|---|---|---|
| ¹ *Propylenethylen-Copolymeres mit 10 Gew.-% Ethylen* ² 190°C/2,16 kg für LDPE und LLDPE und 230°C/2,16 kg für Polypropylen | | | |

Der erhaltene Blasschlauch mit einem Flächengewicht von 10,4 g/m² (entsprechend einer Foliendicke von 11 µm) wurde flachgelegt und an den beiden Seiten aufgeschlitzt, so dass sich zwei Folienbahnen ergaben. Die beiden Ausgangsfolienbahnen wurden gemäß dem erfindungsgemäßen Verfahren einem Heizzylinder zugeführt, wie in Figur 1 mit den Ausgangsfolienbahnen 1 und 2 gezeigt. Die Heizzylinderoberflächentemperatur betrug 130 °C. Dadurch wurden beide Ausgangsfolienbahnen so erwärmt, dass sich jede von ihnen im teilweise schmelzeflüssigen Zustand befand. Anschließend wurde die erhaltene zweilagige Folienbahn einem gekühlten Walzenspalt (wassergekühlt mit 10-15°C) zugeführt. Die Walzen des Kühlwalzenspalts wurden bei einer höheren Bahngeschwindigkeit als die Heizwalze betrieben, so dass die Folienbahn gereckt wurde. Aus der Differenzgeschwindigkeit von Heizwalze zu Kühlwalzenspalt ergibt sich der Reckgrad. Die zweilagige Folienbahn wurde bei drei verschiedenen Reckgraden gereckt, wobei sich folgende Flächengewichte für die Folienbahn ergaben:
- 77 % Reckgrad; Reckverhältnis 1:1,43; Flächengewicht 14,5 g/m² (20,8:1,43);
- 83 % Reckgrad; Reckverhältnis 1:1,72; Flächengewicht 12,1 g/m² (20,8:1,72;)
- 136 % Reckgrad; Reckverhältnis 1:2,08; Flächengewicht 10,0 g/m² (20,8:2,08).

Bei Messung der Folienbahn mit einer CCD-Kamera zeigte die Kamera an, dass die zweilagige Folienbahn 95% weniger Löcher als die einlagige Blasausgangsfolienbahn hatte. Zudem war die Folie in ihren Eigenschaften (beispielsweise Zugfestigkeit, Reißfestigkeit, Reißdehnung, Durchstoßfestigkeit) gegenüber Folien des Standes der Technik gleichwertig bzw. verbessert.

## Patentansprüche

1. Verfahren zur Herstellung einer mehrlagigen Folienbahn aus wenigstens zwei Ausgangsfolienbahnen (1, 2) aus thermoplastischem Polymermaterial, wobei jede Ausgangsfolienbahn wenigstens eine niedrig schmelzende Polymerkomponente und wenigstens eine hoch schmelzende Polymerkomponente umfasst, wobei das Verfahren folgende Schritte umfasst:
Herstellen der wenigstens zwei Ausgangsfolienbahnen (1, 2) durch Blasextrusion,
Cast-Extrusion, oder eine Kombination von Blasextrusion und Cast-Extrusion,
Führen der wenigstens zwei Ausgangsfolienbahnen (1, 2) bis zu deren teilweise schmelzflüssigem Zustand, bei dem sich in jeder Ausgangfolienbahn die wenigstens eine niedrig schmelzende Polymerkomponente im schmelzflüssigen Zustand befindet und die wenigstens eine hoch schmelzende Polymerkomponente nicht im schmelzflüssigen Zustand befindet, zusammen über wenigstens eine Heizwalze (5), und Führen der mehrlagigen, teilweise geschmolzenen Folienbahn durch einen gekühlten Walzenspalt.

2. Verfahren nach Anspruch 1, wobei die wenigstens zwei Ausgangsfolienbahnen gleich sind.

3. Verfahren nach Anspruch 1 oder 2, wobei die wenigstens zwei Ausgangsfolienbahnen zwei durch Blasextrusion hergestellte Ausgangsfolienbahnen sind, wobei ein Blasfolienschlauch hergestellt wird, der Schlauch flachgelegt wird, gegebenenfalls an den beiden Seiten aufgetrennt wird, und wobei die beiden Folienbahnen getrennt oder zusammen der Heizwalze zugeführt werden.

4. Verfahren nach irgendeinem der Ansprüche 1 bis 3, wobei jede Ausgangsfolienbahn 15 bis 85 Gew.-% niedrig schmelzende Polymerkomponente und 85 bis 15 Gew.-% hoch schmelzende Polymerkomponente, bezogen auf 100 Gew.-% niedrig und hoch schmelzende Polymerkomponente, umfasst.

5. Verfahren nach irgendeinem der Ansprüche 1 bis 4, wobei jede Ausgangsfolienbahn wenigstens ein Polyethylen als niedrig schmelzende Polymerkomponente und wenigstens ein Polypropylen als hoch schmelzende Polymerkomponente umfasst.

6. Verfahren nach irgendeinem der Ansprüche 1 bis 5, wobei jede Ausgangsfolienbahn auf 5 bis 20 °C unterhalb des Kristallitschmelzpunktes der wenigstens einen hoch schmelzenden Polymerkomponente erwärmt wird.

7. Verfahren nach irgendeinem der Ansprüche 1 bis 6, wobei die den gekühlten Walzenspalt bildenden Walzen bei einer höheren Geschwindigkeit als die wenigstens eine Heizwalze angetrieben werden.

8. Verfahren nach irgendeinem der Ansprüche 1 bis 7, wobei die mehrlagige Folienbahn zwischen der wenigstens einen Heizwalze und dem gekühlten Walzenspalt gereckt wird, insbesondere bei einem Reckverhältnis von wenigstens 1:1,2, bevorzugt von wenigstens 1:1,5, stärker bevorzugt von wenigstens 1:2.

9. Verfahren nach irgendeinem der Ansprüche 1 bis 8, wobei die mehrlagige Folienbahn im gekühlten Walzenspalt einer Abkühlung auf wenigstens 10 bis 30 °C unterhalb des Kristallitschmelzpunktes der wenigstens einen niedrig schmelzenden Polymerkomponente jeder Ausgangsfolienbahn unterworfen wird.

10. Verfahren nach irgendeinem der Ansprüche 1 bis 9, wobei die Ausgangsfolienbahnen Füllstoff enthalten, insbesondere in einer Menge von 10 bis 90 Gew.-%, bevorzugt 20 bis 80 Gew.-%, bezogen auf jeweils 100 Gew.-% der Ausgangsfolienbahn.

11. Verfahren nach Anspruch 10, wobei wenigstens eine Ausgangsfolienbahn mikroporös ist.

12. Verfahren nach irgendeinem der Ansprüche 1 bis 11, wobei wenigstens eine Ausgangsfolienbahn im Verlauf ihrer Herstellung in Maschinen- oder Querrichtung oder in Maschinen- und Querrichtung gereckt wird.

13. Mehrlagige Folienbahn, erhältlich durch ein Verfahren nach irgendeinem der Ansprüche 1 bis 12.

14. Mehrlagige Folienbahn nach Anspruch 13, mit einem Flächengewicht im Bereich von 1 bis 30 g/m², insbesondere von 5 bis 25 g/m², bevorzugt von 7 bis 20 g/m², stärker bevorzugt von 10 bis 20 g/m².

15. Verwendung der mehrlagigen Folienbahn nach Anspruch 13 oder 14 im Hygiene- oder Medikalbereich, insbesondere für Backsheets in Windeln, für Betteinlagen oder Damenbinden.

## Claims

1. A process for producing a multi-layered film web from at least two starting film webs (1, 2) of thermoplastic polymer material, each starting film web comprising at least one low-melting polymer component and at least one high-melting polymer component, the process comprising the following steps:
producing the at least two starting film webs (1, 2) by blown film extrusion, cast extrusion or a combination of blown film extrusion and cast extrusion,
passing the at least two starting film webs (1, 2) up to their partly molten state, in which in each starting film web, the at least one low-melting polymer component exists in the molten liquid state, and the at least one high-melting polymer component does not exist in the molten liquid state, jointly over at least one heating roller (5), and
passing the multi-layered, partly molten film web through a cooled roller nip.

2. The process according to claim 1, wherein the at least two starting film webs are identical.

3. The process according to claim 1 or 2, wherein the at least two starting film webs are two starting film webs produced by blown film extrusion, wherein a blown film tube is produced, the tube is laid flat, separated at the two sides if applicable, and wherein the two film webs are fed to the heating roller separately or jointly.

4. The process according to any one of claims 1 to 3, wherein each starting film web comprises 15 to 85% by weight of low-melting polymer component and 85 to 15% by weight of high-melting polymer component, based on 100% by weight of low-melting and high-melting polymer components.

5. The process according to any one of claims 1 to 4, wherein each starting film web comprises at least one polyethylene as the low-melting polymer component and at least one polypropylene as the high-melting polymer component.

6. The process according to any one of claims 1 to 5, wherein each starting film web is heated to 5 to 20°C below the crystallite melting point of the at least one high-melting polymer component.

7. The process according to any one of claims 1 to 6, wherein the rollers forming the cooled roller nip are driven at a higher velocity than the at least one heating roller.

8. The process according to any one of claims 1 to 7, wherein the multi-layered film web is stretched between the at least one heating roller and the cooled roller nip, in particular at a stretching ratio of at least 1:1.2, preferably of at least 1:1.5, more preferably of at least 1:2.

9. The process according to any one of claims 1 to 8, wherein the multi-layered film web is subjected to cooling in the cooled roller nip to at least 10 to 30°C below the crystallite melting point of the at least one low-melting polymer component of each starting film web.

10. The process according to any one of claims 1 to 9, wherein the starting film webs contain filler, in particular in an amount of 10% to 90% by weight, preferably 20% to 80% by weight, each based on 100% by weight of the starting film web.

11. The process according to claim 10, wherein at least one starting film web is microporous.

12. The process according to any one of claims 1 to 11, wherein at least one starting film web is, in the course of its production, stretched in the machine or transverse direction or in the machine and transverse direction.

13. Multi-layered film web, obtainable by a process according to any one of claims 1 to 12.

14. Multi-layered film web according to claim 13, having a basis weight within the range of from 1 to 30 g/m², in particular from 5 to 25 g/m², preferably from 7 to 20 g/m², more preferably from 10 to 20 g/m².

15. Use of the multi-layered film web according to claim 13 or 14 in the hygiene or medical field, in particular for back sheets of diapers, for mattress protectors or sanitary napkins.

## Revendications

1. Procédé de fabrication d'une feuille continue multicouche à partir d'au moins deux feuilles continues de départ (1, 2) en un matériau polymère thermoplastique, dans lequel chaque feuille continue de départ comprend au moins un composant polymère à bas point de fusion et au moins un composant polymère à haut point de fusion, le procédé comprenant les étapes suivantes :
fabrication des au moins deux feuilles continues de départ (1, 2) par extrusion-soufflage, par extrusion-coulée ou par une combinaison d'un extrusion-soufflage et d'une extrusion-coulée,
envoi sur au moins un cylindre chauffant (5) des au moins deux feuilles continues de départ (1, 2) jusqu'à leur état partiellement fluide, dans lequel dans chaque feuille continue de départ l'au moins un composant polymère à bas point de fusion se présente à l'état fluide et l'au moins un composant polymère à haut point de fusion ne se présente à l'état fluide, et
envoi de la feuille continue multicouche partiellement fondue dans l'espace refroidi entre les cylindres.

2. Procédé selon la revendication 1, dans lequel les au moins deux feuilles continues de départ sont identiques.

3. Procédé selon la revendication 1 ou 2, dans lequel les au moins deux feuilles continues de départ sont deux feuilles continues de départ fabriquées par extrusion-soufflage ; on fabrique un film tubulaire soufflé, on dépose le film tubulaire à plat, on le sépare éventuellement sur ses deux faces ; et les deux feuilles continues sont amenées séparément ou ensemble au cylindre chauffant.

4. Procédé selon l'une des revendications 1 à 3, dans lequel chaque feuille continue de départ comprend 15 à 85 % en poids du composant polymère à bas point de fusion et 85 à 15 % en poids du composant polymère à haut point de fusion, pour 100 % en poids de la somme des composants polymères à bas et à haut point de fusion.

5. Procédé selon l'une des revendications 1 à 4, dans lequel chaque feuille continue de départ comprend au moins un polyéthylène en tant que composant polymère à bas point de fusion et au moins un polypropylène en tant que composant polymère à haut point de fusion.

6. Procédé selon l'une des revendications 1 à 5, dans lequel chaque feuille continue de départ est chauffée à une température de 5 à 25 °C inférieure au point de fusion des cristallites de l'au moins un composant polymère à haut point de fusion.

7. Procédé selon l'une des revendications 1 à 6, dans lequel les cylindres entre lesquels est formé l'écartement refroidi entre les cylindres sont entraînés à une vitesse plus grande que l'au moins un cylindre chauffant.

8. Procédé selon l'une des revendications 1 à 7, dans lequel la feuille continue multicouche est étirée entre l'au moins un cylindre chauffant et l'écartement refroidi entre les cylindres, en particulier pour un taux d'étirage d'au moins 1:1,2, de préférence d'au moins 1:1,5, plus préférentiellement d'au moins 1:2.

9. Procédé selon l'une des revendications 1 à 8, dans lequel la feuille continue multicouche est, dans l'écartement refroidi entre les cylindres, soumis à un refroidissement à une température d'au moins 10 à 30 °C inférieure au point de fusion des cristallites de l'au moins un composant polymère à bas point de fusion de chaque feuille continue de départ.

10. Procédé selon l'une des revendications 1 à 9, dans lequel les feuilles continues de départ contiennent une charge, en particulier en une quantité de 10 à 90 % en poids, de préférence de 20 à 80 % en poids, dans chaque cas pour 100 % en poids de la feuille continue de départ.

11. Procédé selon la revendication 10, dans lequel au moins une feuille continue de départ est microporeuse.

12. Procédé selon l'une des revendications 1 à 11, dans lequel au moins une feuille continue de départ est étirée au cours de sa fabrication dans le sens machine ou travers, ou dans les sens machine et travers.

13. Feuille continue multicouche pouvant être obtenue par un procédé selon l'une des revendications 1 à 12.

14. Feuille continue multicouche selon la revendication 13, présentant une masse surfacique comprise dans la plage de 1 à 30 g/m², en particulier de 5 à 25 g/m², de préférence de 7 à 20 g/m², plus préférentiellement de 10 à 20 g/m².

15. Utilisation de la feuille continue multicouche selon la revendication 13 ou 14 dans le domaine hygiénique ou médical, en particulier pour des enveloppes extérieures de couches pour bébé, pour des alèses ou des serviettes hygiéniques.
